# EUROPEAN PATENT APPLICATION

(11) **EP 3 292 863 A1**
(43) Date of publication of application: **14.03.2018**
(21) Application number: 17020395.4
(22) Date of filing: 31.08.2017
(51) Int. Cl.: A61K 9/20, A61K 31/7072

(54) **SOLID PHARMACEUTICAL DOSAGE FORM COMPRISING SOFOSBUVIR**

(30) Priority: 09.09.2016 CZ 20160553
(71) Applicant: Zentiva, k.s., 102 37 Praha 10 (CZ)
(72) Inventor: Sedmak, Gregor, 102 00 Praha 10 (CZ); Obadalova, Iva, 169 00 Praha 6 (CZ)
(74) Representative: Jirotkova, Ivana

(57) **Abstract**

The present subject relates to the solid pharmaceutical dosage form comprising 1-Methylethyl N-[(S)-{[(2R,3R,4R,5R)-5-(2,4-dioxo-3,4-dihydropyrimidin-1(2H)-yl)-4-fluoro-3-hydroxy-4-methyltetrahydrofuran-2-yl]methoxy}phenoxyphosphoryl]-l-alaninate known also as sofosbuvir, and a process of preparation of the pharmaceutical dosage form according to the invention.

## Description

### Field of the Invention

The present invention relates to the solid pharmaceutical dosage form comprising 1-Methylethyl N-[(S)-{[(2R,3R,4R,5R)-5-(2,4-dioxo-3,4-dihydropyrimidin-1 (2H)-yl)-4-fluoro-3-hydroxy-4-methyltetrahydrofuran-2-yl]methoxy}phenoxyphosphoryl]-I-alaninate known also as sofosbuvir, and a process of preparation of the pharmaceutical dosage form according to the invention.

### Background Art

WO 2013/082003 discloses that the tablets comprising sofosbuvir may be prepared according to methods known in the art, including dry granulation, wet granulation and direct compression. However, it has been found that dry granulation is particularly suitable method.

It is known that the sofosbuvir is a deliquescent moiety. This is the reason why high amount of fillers are needed in order to prevent sticking of material to the manufacturing equipment, namely to the rollers during roller compaction process and tableting tools during tableting process. Therefore, if the composition comprising sofosbuvir contains 400 mg of sofosbuvir which represents 33.33% of the total composition as stated in WO 2013/082003, than the total tablet weight may be calculated to be 1200 mg. This tablet weight, however, is very high and therefore represents a problematic administration for patients.

WO 2015/150561 discloses solid compositions comprising sofosbuvir in amorphous form, wherein at least 99 weight-% of the solid composition consist of the sofosbuvir and the at least one matrix compound, and wherein the solid composition contains the sofosbuvir in an amount of at least 55 weight-% based on the combined weight of the sofosbuvir and the at least one matrix compound.

According to WO 2015/150561, some of the disclosed matrix compounds useful for the preparation of the composition comprising sofosbuvir are silicon-based inorganic adsorbents like Syloid® 72 FP, Syloid® 244 FP, Neusilin® UFL2 and Neusilin® US2. However, if the at least 99 weight-% of the solid composition consist of complex of the sofosbuvir and one matrix compound, it is very difficult to prepare a tablet with useful tableting properties. There is a need to add to the composition some tableting aids, like a reasonable amount of binder, filler, disintegrant and lubricant, otherwise it is not possible to prepare a tablet.

In addition, WO 2015/150561 discloses methods of preparation of the sofosbuvir in which at least 99 weight-% of the solid composition consist of the sofosbuvir and the at least one matrix compound. This method consists of dissolving of sofosbuvir in a solvent obtaining a solution comprising the sofosbuvir and a subsequent drying. Specifically, two methods are disclosed in WO 2015/150561, namely lyophilization and spray-drying. Both processes, however, are very rarely found in the usual pharmaceutical manufacturing plants. Therefore, there is a high need to provide a convenient manufacturing process of a composition comprising sofosbuvir, which is stable, not deliquescent, has good tableting properties, is fast dissolving and at the same time convenient for patient administration.

### Detailed Description of the Invention

After an extensive work it has been found that it is possible to prepare a stable, not deliquescent, fast dissolving tablet composition comprising sofosbuvir with good tableting properties and convenient for patient administration.

In one aspect of the present invention, composition comprising sofosbuvir consists of sofosbuvir, inorganic silicon based filler and further excipients.

In another aspect of the present invention, it has been surprisingly found that to the contrary of teaching of prior art, it is not necessary to prepare a solid composition comprising sofosbuvir by the non-typical lyophilization or spray-drying process.

One of the simple and readily available manufacturing process is the fluid bed granulation method, which is sufficient for producing a stable, not deliquescent, fast dissolving tablet composition comprising sofosbuvir with good tableting properties and convenient for patient administration.

The composition comprising sofosbuvir may be prepared by the following standard fluid bed process:
a) dissolving sofosbuvir in a suitable solvent,
b) charging inorganic silicon based filler into the fluid bed granulator,
c) spraying the dissolved sofosbuvir onto the inorganic silicon based filler in a fluidized mode of operation of fluid bed granulation machine,
d) drying of the obtained granulate,
e) any further standard processing steps may optionally be employed to finish the preparation of the final composition consisting of sofosbuvir.

According to the present invention, in case of a fluid bed granulation method, any kind of a suitable drying process in step d) from above may be employed. Typically, the granulate is dried into the same fluid bed granulation machine via fluidization of obtained granules. Optionally, a second granulate drying stage may additionally be employed. For the optional second drying stage, a standard tray drier, optionally employing a vacuum may be used in order to additionally decrease the amount of residual solvents in the granulate.

Another simple and readily available manufacturing process is the batch dryer. One of the non-binding examples of a batch dryer is a single pot processor. Typically, this kind of a batch dryer dries the organic solvent by employing the vacuum, which may be assisted by gas-stripping or also microwave technology or a combination thereof. By employing batch dryer, it is possible to produce a stable, not deliquescent, fast dissolving tablet composition comprising sofosbuvir with good tableting properties and convenient for patient administration.

Batch dryer can be employed in the following way in order to manufacture the composition comprising sofosbuvir in the following way:
a) dissolving sofosbuvir in a suitable solvent and adding the inorganic silicon based filler in the batch dryer,
b) drying the obtained wet material either by employing the vacuum, optionally employing gas stripping, or by microwaves, or by a combination thereof, in order to obtain a granulate
c) any further standard processing steps may optionally be employed to finish the preparation of the final composition consisting of sofosbuvir

According to the present invention, any kind of a suitable solvent may be used for dissolving sofosbuvir. A suitable solvent may be selected from, but not limited to water, ethyl acetate, ethanol, methanol, acetone, isopropanol and mixtures thereof. Particularly preferred are methanol and acetone.

The composition comprising sofosbuvir typically comprises inorganic silicon based filler. Suitable inorganic silicon based fillers may be selected from mesoporous silica gels with internal 3-dimensional network of primary particles, colloidal silicon dioxide without the extensive internal 3-dimensional network structure as well as mixed silicon-aluminum-magnesium silicates. Particularly preferred are colloidal silicon dioxide of Aeroperl® type and mesoporous silica gels of Syloid® type.

The composition comprising sofosbuvir also typically comprises inorganic further excipients. Suitable further excipients may be selected from a second filler, binder, disintegrant, lubricant and coating agents. A suitable second filler may be selected from, but not limited to water soluble fillers like oligosaccharides, monosaccharides and sugar alcohols like fructose, glucose, saccharose, lactose anhydrous, raffinose, isomalt, dextrates, mannitol, trehalose, erythritol, sorbitol, maltitol, lactose monohydrate, lactose anhydrous, xylitol and lactitol and water insoluble fillers like calcium hydrogen phosphate anhydrous, calcium hydrogen phosphate dihydrate, microcrystalline cellulose and mixtures thereof. A suitable binder may be selected from, but not limited to maltose, starch, pregelatinized starch, polymethacrylates, copovidone, povidone, cellulose powder, crystalline cellulose, microcrystalline cellulose, siliconized microcrystalline cellulose, cellulose derivatives such as hydroxymethylcellulose, hydroxyethylcellulose, hydroxypropylcellulose and hydroxypropylmethylcellulose, and mixtures thereof. A suitable disintegrant may be selected from, but not limited to crospovidone, croscarmellose sodium, hydroxypropyl starch, low-substituted hydroxypropyl cellulose, sodium starch glycolate and mixtures thereof. A suitable lubricant may be selected from, but not limited to magnesium stearate, hydrogenated castor oil, talc, sodium stearyl fumarate, hydrogenated vegetable oil, magnesium trisilicate and mixtures thereof. A suitable coating agents may be selected from, but not limited to hypromellose, hydroxypropylcellulose, polyvinyl alcohol, polyethylene glycol and mixtures thereof.

In another aspect of the present invention, composition comprising sofosbuvir consists of sofosbuvir in an amount of 35 - 50 w/w-%, inorganic silicon based filler in an amount of 35 - 50 w/w-% and further excipients in such a way that the total sum of further excipients equals to 100 w/w-% in a final dosage form. Preferred are compositions in which sofosbuvir : inorganic silicon based filler is between 45:55 to 55:45 w/w ratio and the sum of further excipients equals to 100 w/w-% in a final dosage form. Particularly preferred are compositions in which sofosbuvir : inorganic silicon based filler is in a 1:1 w/w ratio, a dried mixture of sofosbuvir : inorganic silicon based filler comprises from 70 w/w to 97? w/w of the final dosage form composition and the sum of further excipients equals to 100 w/w-%. In the even more preferred composition, composition comprising sofosbuvir consists of sofosbuvir in an amount of about 400 mg, inorganic silicon based filler in an amount of 400 mg and further excipients in the total amount between 20 and 400 mg. In the most preferred composition, composition comprising sofosbuvir consists of sofosbuvir in an amount of about 400 mg, inorganic silicon based filler in an amount of 400 mg and further excipients in the total amount between 50 and 200 mg.
The tablets prepared by the above described process and having the optimize composition exhibits complying properties i.e. good hardness, negligible friability and fast dissolution rate. Especially proved tablet model was having no second filler, containing 400 mg of sofosbuvir, 400 mg of the inorganic silica and 40 to 60 mg of the other excipients composed with a disintegrant and lubricant. Another especially preferred tablets model contains 400 mg of sofosbuvir 400 mg of the inorganic silica and 150 to 200 mg of the further excipients. The tablet contains the second filer selected from the soluble sugars and sugar alcohol. Mannitol and anhydrous lactose can be named as an example of the second fillers in this embodiment.

### Example 1

The example formulations are prepared by the conventional fluid bed granulation. Sofosbuvir is dissolved in methanol. Inorganic silicon based filler is charged into the fluid bed granulation machine. Dissolved sofosbuvir is sprayed onto the inorganic silicon based filler in a fluidized mode of operation of fluid bed granulation machine. The obtained granulate is dried. To the obtained granulate, a second filler, disintegrant and a lubricant are admixed in order to obtain a mixture ready for tableting. Mixture ready for tableting is tableted on a high speed tableting machine in order to obtain tablets comprising sofosbuvir.

| | | 1A | 1B | 1C | 1D |
|---|---|---|---|---|---|
| Sofosbuvir | active | 400.00 | 400.00 | 400.00 | 400.00 |
| Colloidal silicon dioxide | inorganic silicon based filler | 400.00 | | 400.00 | |
| Mesoporous silica gel | inorganic silicon based filler | | 400.00 | | 400.00 |
| Lactose anhydrous | second filler | | | 110.00 | |
| Mannitol | second filler | | | | 70.00 |
| Calcium hydrogen phosphate anhydrous | second filler | | | 60.00 | 100.00 |
| Crospovidone | disintegrant | 40.00 | 40.00 | 20.00 | 20.00 |
| Magnesium stearate | lubricant | 10.00 | | 10.00 | |
| Sodium stearyl fumarate | lubricant | | 10.00 | | 10.00 |
| Total | | 850.00 | 850.00 | 1000.00 | 1000.00 |

### Example 2

The example formulations are prepared by the conventional batch dryer. Sofosbuvir is dissolved in acetone. Solution is placed into the batch dryer. Inorganic silicon based filler is added and mixed. Acetone is dried by a vacuum, and thus granules containing sofosbuvir / inorganic silicon based filler are obtained. To the obtained granulate, second filler, disintegrant and a lubricant are admixed in order to obtain a mixture ready for tableting. Mixture ready for tableting is tableted on a high speed tableting machine in order to obtain tablets comprising sofosbuvir.

| | | 2A | 2B | 2C | 2D | 2E |
|---|---|---|---|---|---|---|
| Sofosbuvir | active | 400 | 400 | 400 | 400 | 400 |
| Colloidal silicon dioxide | inorganic silicon based filler | 400 | 400 | 400 | | |
| Mesoporous silica gel | inorganic silicon based filler | | | | 400 | 400 |
| Mannitol | second filler | | | 150 | | 100 |
| Calcium hydrogen phosphate anhydrous | second filler | | | | 60 | |
| Microcrystalline cellulose | second filler | | | | 50 | |
| Crospovidone | disintegrant | 10 | 40 | 30 | 30 | 30 |
| Magnesium stearate | lubricant | 10 | | | 10 | |
| Sodium stearyl fumarate | lubricant | | 10 | 10 | | 10 |
| Total | | 820 | 850 | 990 | 950 | 940 |

## Claims

1. A pharmaceutical solid composition in the compressed form comprising sofosbuvir and an inorganic silicon based filler, which is selected from mesoporous silica gels with internal 3-dimensional network of primary particles, colloidal silicon dioxide without the extensive internal 3-dimensional network structure or mixed silicon-aluminum-magnesium silicates, said composition containing either
only one filler, lubricant and disintegrant, or
one or more further fillers.

2. The composition according to claim 1, in which the further fillers are selected from water soluble or water insoluble ones or a combination thereof.

3. The composition according to claims 1 or 2, in which the water soluble fillers are selected from oligosaccharides, monosaccharides and sugar alcohols.

4. The composition according to claims 1 or 2, in which the water insoluble fillers are selected from calcium hydrogen phosphate anhydrous or dihydrate, and microcrystalline cellulose.

5. The composition according to claims 1 to 4, in which the fillers are combined water soluble and water insoluble ones.

6. The composition according to claim 5, in which the further fillers are the combination of mannitol or/and lactose anhydrous and calcium hydrogen phosphate anhydrous.

7. The composition according to any one of the preceding claims, which has a total weight between 850 to 1200 mg.

8. The composition according to any one of the preceding claims, in which the ratio of sofosbuvir : inorganic silicon based filler is between 45:55 to 55:45 w/w.

9. The composition according to claim 8, in which the ratio of sofosbuvir :
inorganic silicon based filler is between 49 : 51 to 51 : 49.

10. The composition according to claim 9, in which the ratio of sofosbuvir :
inorganic silicon based filler is 50 : 50.

11. A method of manufacturing the composition according to any one of the preceding claims, the method comprising the following standard fluid bed process:
a) dissolving sofosbuvir in a suitable solvent,
b) charging inorganic silicon based filler into the fluid bed granulator,
c) spraying the dissolved sofosbuvir onto the inorganic silicon based filler in a fluidized mode of operation of fluid bed granulation machine,
d) drying of the obtained granulate,
e) and optionally employing any further standard processing steps to finish the preparation of the final composition consisting of sofosbuvir.

12. A method of manufacturing the composition according to any one of the preceding claims, using a batch dryer, comprising the following steps:
a) dissolving sofosbuvir in a suitable solvent and adding the inorganic silicon based filler in the batch dryer,
b) drying the obtained wet material either by employing the vacuum, optionally employing gas stripping, or by microwaves, or by a combination thereof, in order to obtain a granulate,
c) and optionally employing any further standard processing steps to finish the preparation of the final composition consisting of sofosbuvir.

13. The method according to claim 11 or 12, in which the suitable solvent is selected from water, ethyl acetate, ethanol, methanol, acetone, isopropanol and mixtures thereof.

14. The method according to claim 13, in which the solvent is selected from methanol, acetone and mixtures thereof.
